# EUROPÄISCHE PATENTSCHRIFT

(11) **EP 1 632 256 B1**
(45) Veröffentlichungstag und Bekanntmachung des Hinweises auf die Patenterteilung: **30.09.2009**
(21) Anmeldenummer: 05107617.2
(22) Anmeldetag: 19.08.2005
(51) Int. Cl.: A61L 31/02, A61L 31/14

(54) **Endoprothese aus einer Magnesiumlegierung**
Endoprothesis made of a magnesium alloy
Endoprothèse à base d'un alliage de magnésium

(30) Priorität: 07.09.2004 DE 102004432317
(43) Veröffentlichungstag der Anmeldung: 08.03.2006
(73) Patentinhaber: Biotronik VI Patent AG, 6340 Baar (CH)
(72) Erfinder: Harder, Claus, 91080, Uttenreuth (DE); Kuttler, Marc, 12205, Berlin (DE); Gerold, Bodo, 91225 Zellingen (DE)
(74) Vertreter: Lindner-Vogt, Karin L.

(56) Entgegenhaltungen:
- EP-A- 1 419 793
- EP-A- 1 552 856
- DE-A1- 10 128 100
- STAIGER M.P. ET AL: "Magnesium and its alloys as orthopedic biomaterials: a review" BIOMATERIALS, Bd. 27, 24. November 2005 (2005-11-24), Seiten 1728-1734, XP002469482
- MANI G. ET AL: "Coronary stents: a materials perspective" BIOMATERIALS, Bd. 28, 22. Dezember 2006 (2006-12-22), Seiten 1689-1710, XP002469483

## Beschreibung

Die Erfindung betrifft eine Endoprothese, insbesondere eine intraluminale Endoprothese wie einen Stent, mit einer Tragstruktur, die ganz oder in Teilen aus einer Magnesiumlegierung besteht.

### Stand der Technik

Viele Endoprothesen haben den Zweck, im Inneren des Körpers eines Patienten eine Stützfunktion zu übernehmen. Dementsprechend sind Endoprothesen implantierbar ausgeführt und besitzen eine Tragstruktur, die die Stützfunktion gewährleistet. Bekannt sind Implantate aus metallischen Werkstoffen. Die Wahl von Metallen als Werkstoff für die Tragstruktur eines derartigen Implantats beruht vor allem auf den mechanischen Eigenschaften von Metallen.

Metallische Stents sind in großer Zahl bekannt. Einer der Hauptanwendungsbereiche solcher Stents ist das dauerhafte Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bieten. Derartige Stents besitzen in der Regel eine Umfangswandung von ausreichender Tragkraft, um das verengte Gefäß im gewünschten Maße offen zu halten. Um einen ungehinderten Blutfluss durch den Stent zu ermöglichen, ist diese an beiden Stirnseiten offen. Kompliziertere Ausführungen ermöglichen auch einen ungehinderten Blutfluss in Nebengefäße (Side Branch Access). Die tragende Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. An den Stent wird daher grundsätzlich die Anforderung gestellt, dass seine Tragstruktur im aufgeweiteten Zustand eine ausreichende Tragkraft aufweist, um das Gefäß offen zu halten. Um unnötige Gefäßbeschädigungen zu vermeiden, ist außerdem gewünscht, dass der Stent nach dem Aufweiten und nach Entfernen des Ballons nur wenig elastisch zurückfedert (Recoil), so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt.

In einigen Fällen, wie z. B. bei Verschraubungen von komplizierten Brüchen oder anderen Verbindungs- und Stützelementen in der Knochenchirurgie, Nahtmaterialien oder insbesondere im Falle intraluminaler Endoprothesen wie Stents, ist eine dauerhafte Halte- und Stützfunktion durch die Endoprothese nicht erforderlich. Vielmehr kann sich in einigen dieser Anwendungsfälle das Körpergewebe unter Anwesenheit der Halte- und Stützprothese derart erholen, dass eine dauerhafte Stützwirkung durch die Prothese nicht erforderlich ist. Dies hat zu dem Gedanken geführt, solche Prothesen aus bioresorbierbarem Material zu fertigen.

In einigen Fällen, insbesondere im Falle solcher intraluminaler Endoprothesen wie Stents, ist eine dauerhafte Stützfunktion durch die Endoprothese nicht erforderlich. Vielmehr kann sich in einigen dieser Anwendungsfälle das Körpergewebe unter Anwesenheit der Stützprothese derart erholen, dass eine dauerhafte Stützwirkung durch die Prothese nicht erforderlich ist. Dies hat zu dem Gedanken geführt, solche Prothesen aus bioresorbierbarem Material zu fertigen.

Neben den gewünschten mechanischen Eigenschaften eines Stents soll dieser möglichst in einer Weise mit dem Körpergewebe am Implantationsort interagieren, dass es nicht zu erneuten Gefäßverengungen, insbesondere durch den Stent selbst hervorgerufenen Gefäßverengungen, kommt. Eine Restenose (Wiederverengung des Gefäßes) soll möglichst vermieden werden. Auch ist es wünschenswert, wenn von dem Stent möglichst gar keine oder nur eine sehr geringe inflammatorische Wirkung ausgeht. In Bezug auf einen biodegradierbaren Metallstent ist es darüber hinaus wünschenswert, wenn von den Abbauprodukten des Metallstents möglichst keine oder nur sehr geringe negative physiologische Wirkungen ausgehen, bzw. sogar positive physiologische Wirkungen bedingt sind.

Aus der DE 197 31 021 ist ein bioresorbierbarer Metallstent bekannt, dessen Werkstoff als Hauptbestandteil Magnesium, Eisen oder Zink enthält. Aufgrund der mechanischen Eigenschaften, dem Degradationverhalten und der Biokompatibilität sind insbesondere Magnesiumlegierungen zu präferieren.

In DE 102 53 634, DE 101 28 100 oder EP 1 395 297 wird auf den Einsatz von solchen biodegradierbaren Magnesiumlegierungen zu medizinischen Zwecken, wie Platten, Schrauben, Nahtmaterial oder Stents fokussiert. Die Magnesiumlegierungen weisen einen Magnesiumgehalt von über 70 Gew.% bzw. über 90 Gew.% auf. Mit steigendem Magnesiumgehalt nimmt aber die Degradationszeit und damit der Zeitraum der benötigten mechanischen Integrität schnell ab. Bei den Stentanwendungen liegen die Degradationszeiträume solche Legierungen typisch deutlich unter 30 Tagen. Dies ist für viele Anwendungen in der Medizin nicht ausreichend. So ist bei Stentanwendungen bislang ungeklärt, wie lange die mechanische Integrität für eine ausreichende Stützfunktion erforderlich ist. Schätzungen von Experten variieren von wenigen Tagen bis hin zu einem Jahr. Bei komplizierten Brüchen kann der Heilungsprozess leicht 6 Monate betragen.

Auch die mechanische Stabilität ist insbesondere für Endoprothese-Anwendungen äußerst wichtig. Sie ermöglicht eine kompakte Auslegung der Endoprothese bei ausreichender Stabilität. Bei Stents z. B. wird versucht immer kleinere Stegbreiten zu verwirklichen, da Studien belegt haben, dass mit der Stegbreite u. a. das Restenoserisiko deutlich reduziert wird, da der Steg das umliegende Gewebe u. a. mechanisch reizt. Um dies zu erreichen sind entsprechende Materialfestigkeiten erforderlich. Bisher verwendete Magnesiumlegierungen, wie in den Schriften DE 102 53 634, DE 101 28 100 oder EP 1 395 297 aufgeführt, sind relativ weich. Dies schränkt den Einsatzbereich als Endoprothese ein.

Neben den mechanischen Eigenschaften ist die Biokompatibilität der verwendeten Legierung essentiell für einen Einsatz als medizinisches Implantat. Alternative biodegradierbare Materialien, wie z. B. Polymere, haben neben den schlechten mechanischen Eigenschaften insbesondere mit der Schwierigkeit einer geringen Biokompatibilität zu kämpfen. Magnesiumlegierungen haben bereits deutlich bessere Eigenschaften belegt, allerdings handelt es sich dabei vor allem um aluminiumhaltige Legierungen wie z. B. in den Schriften DE 101 28 100 oder EP 1 395 297 beschrieben. Das Aluminium wird dabei u. a. für die Ausbildung von Deckschichten benötigt, die die Diffusion des Magnesiums und damit den Degradationsprozess verlangsamen sollen. Dies ist gemäß diesen Schriften u. a. erforderlich, um eine ausreichend lange mechanische Stabilität der Endoprothese zu erreichen und Gasausscheidungen beim Degradationsprozess zu verhindern.

Aluminium ist aber dafür bekannt, gesundheitliche Schäden zu verursachen, insbesondere wenn es in ionischer Form vorliegt. So ist Aluminium u. a. dafür bekannt Schäden am Zentralnervensystem zu verursachen und Symptome wie Demenz, Gedächtnisverlust, Antriebslosigkeit oder heftiges Zittern auszulösen. Aluminium gilt als Risikofaktor für die Alzheimer-Krankheit (Harold D. Foster Ph. D., Journal für Orthomolekulare Medizin 2/01). Negative Effekte hinsichtlich der Biokompatibilität in unmittelbarer Umgebung von Endoprothesen aus aluminiumhaltigen Magnesiumlegierungen konnten in Experimenten nachgewiesen werden. So wurden in Tierexperimenten pathologische Höfe um degradierende Stege solcher Stents sowie eine ausgeprägte Neointimahyperplasie beobachtet, die dem eigentlichen Zweck des Stents, den Gefäßverschluss zu verhindern, entgegenwirkt. Der Einsatz von Aluminium in degradierbaren medizinischen Implantaten wie insbesondere den Stents ist somit nicht zu favorisieren.

Generell vernachlässigt wurde bislang der Ansatz bei medizinischen Implantaten, körpereigene Heilungsprozesse im Rahmen der Anwendung von Endoprothesen zu aktivieren, um so den Heilungsprozess weiter zu verbessern.

Vor diesem Hintergrund liegt der vorliegenden Erfindung die Aufgabe zugrunde, eine biodegradierbare Endoprothese auf der Basis einer Magnesiumlegierung bereit zu stellen, die die geschilderten Nachteile des Standes der Technik vermeidet. Insbesondere sollen Legierungen mit erhöhter mechanischer Stabilität und längeren Degradationszeiträumen zur Verfügung gestellt werden.

Erfindungsgemäß wird diese Aufgabe durch eine Endoprothese nach Anspruch 1 gelöst. Die Endoprothese weist eine Tragstruktur auf, die ganz oder in Teilen aus einer Magnesiumlegierung folgender Zusammensetzung besteht:
· Magnesium 60.0 bis 88.0 Gew.%,
· Seltenerdmetalle 2.0 bis 30.0 Gew.%,
· Yttrium 2.0 bis 20.0 Gew.%,
· Zirkonium 0.5 bis 5.0 Gew.%,
· Rest 0 bis 10.0 Gew.%,
wobei sich die Legierungskomponenten auf 100 Gew.% addieren. Die Legierung zeigt sehr günstige mechanische, aber auch physiologische Eigenschaften und ein gegenüber den bekannten Legierungen verzögertes Degradationsverhalten in vivo. Sie kann einfach verarbeitet werden und zeigt in ersten Studien einen positiven physiologischen Effekt auf das umgebene Gewebe in Mensch und Tier, wenn die Legierung in Endoprothesen,'insbesondere Stents, verwendet wird.

Die Sammelbezeichnung ,Seltenerdmetall' steht für die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide bezeichnet werden. Der Anteil der Seltenerdmetalle an der Magnesiumlegierung umfasst somit auch den Anteil des Neodyms. Letzterer Anteil ist - sofern vorhanden - ebenfalls auf das Gesamtgewicht der Legierung bezogen und muss im angegebenen Bereich liegen. Liegt der Anteil an Neodym in der Legierung z. B. bei 2.0 Gew.% und der Anteil an Seltenerdmetallen bei 2.5 Gew.%, so werden zwangsläufig Seltenerdmetalle außer Neodym einen Gewichtsanteil an der Legierung von 0.5 Gew.% aufweisen.

Besonders bevorzugt ist eine Legierung mit einem Magnesiumanteil von 60.0 bis 70.0 Gew.%. Die Legierung zeigt ein gegenüber dem Stand der Technik verzögertes Degradationsverhalten, ist aber noch hinreichend biodegradierbar. Die Legierung und die Abbauprodukte zeigten eine gute biologische Verträglichkeit, d. h. es traten in ersten Tests keine immunologischen Reaktionen oder Entzündungen auf.

Der Rest enthält vorzugsweise nur die aus dem Herstellungsprozess der Magnesiumlegierung bedingten Verunreinigungen. Mit anderen Worten, es sind lediglich spezifische Verunreinigungen enthalten, die sich bei der Herstellung der Legierung nicht vermeiden lassen oder Restkomponenten, die bewusst der Legierung zugesetzt werden. Hierdurch werden die positiven physiologischen Effekte und die mechanischen Eigenschaften des Werkstoffs sichergestellt und teils gar erst erreicht.

Ergänzend oder alternativ zu vorgenannter bevorzugter Variante enthält der Rest kein oder allenfalls < 0.01 Gew.% Aluminium. Gerade Aluminium hat einen ausgeprägten negativen Einfluss auf das physiologische Verhalten, wie Materialuntersuchungen sowie in vivo und in vitro Untersuchungen gezeigt haben.

Aufgrund der negativen Eigenschaften, insbesondere auf die Biokompatibilität, werden in den Legierungen neben dem Element Aluminium (AI) vorzugsweise auch die Elemente Kupfer (Cu), Nickel (Ni), Silber (Ag), Quecksilber (Hg), Cadmium (Cd), Beryllium (Be) oder Chrom (Cr) vermieden, d. h. die Elemente sind abgesehen von herstellungsbedingten Verunreinigungen nicht in der Legierung enthalten. Der als Rest bezeichnete Anteil der Legierung beinhaltet vorrangig Massenanteile eines, mehrerer oder aller genannter Elemente unter den folgenden Grenzen:
· Aluminium < 0.01 Gew.%,
· Kupfer < 0.03 Gew.%,
· Nickel < 0.005 Gew.%,
· Silber < 0.01 Gew.%,
· Quecksilber < 0.03 Gew.%,
· Cadmium < 0.03 Gew.%,
· Beryllium < 0.03 Gew.%,
· Chrom < 0.03 Gew.%.

Die Vermeidung dieser Elemente ist für den Zweck der Erfindung von Bedeutung, da diese gesundheitsschädliche Wirkung haben, die mechanischen Eigenschaften der Legierung unerwünscht beeinflussen sowie die auf den Heilungsprozess positiven Einflüsse der Legierung und insbesondere des Magnesiums beeinträchtigen. Bekanntlich können schon geringe Spuren von Verunreinigungen einen metallurgisch und/oder physiologisch erheblichen Effekt haben. Das Identifizieren der Störelemente und insbesondere das Festlegen von Grenzwerten für diese Störelemente liefert damit einen erheblichen technischen Beitrag zur Optimierung der Produkte.

Bevorzugt ist hingegen, dass der Rest eines oder mehrere Elemente aus der Gruppe Lithium, Zink, Eisen, Titan, Tantal, Molybdän und Wolfram enthält. Der Anteil der Komponenten an der Legierung liegt vorzugsweise bei 0.1 bis 5.0 Gew.%, wobei sich deren kumulierter Gesamtanteil auf maximal 10.0 Gew.% addiert. Die Anwesenheit dieser Elemente hat offenbar positiven Einfluss auf das Degradationsverhalten, die mechanischen Eigenschaften und die Biokompatibilität des Implantats.

Tantal, Molybdän und Wolfram werden u. a. für eine verbesserte mechanische Stabilität verantwortlich gemacht. Des Weiteren wird die Röntgensichtbarkeit der Legierung verbessert. Auch Titan, das als äußerst biokompatibel bekannt ist, hat deutlichen Einfluss auf die mechanische Stabilität.

Zink und Eisen können, wie das Magnesium, durch ihr Korrosionsverhalten und grundsätzliche Bedeutung bei den Stoffwechselprozessen im Körper als biodegradierbar bezeichnet werden. Über diese Elemente lässt sich u. a. das Korrosionsverhalten und die Korrosionsgeschwindigkeit beeinflussen.

Ferner ist bevorzugt, dass der Anteil an Gadolinium 2.0 bis 30.0 Gew.%, und - unabhängig davon - Neodym 0.5 bis 10.0 Gew.%, insbesondere 2.0 bis 2.5 Gew.% an der Legierung beträgt. Hierdurch lässt sich die physiologische Verträglichkeit der Legierung und seiner Abbauprodukte noch steigern sowie das Degradationsverhalten für die geplanten Zwecke optimieren.

Mit der hier beschriebenen Magnesiumlegierung konnte ein signifikant verbesserter Degradationsablauf mit deutlich besserer Reproduzierbarkeit erzielt werden, als bisher z. B. für aluminiumhaltige Magnesiumlegierungen bekannt ist (Heart (2003) 89, 651-656). Insbesondere die Reproduzierbarkeit des Degradationsverlaufes ist für eine medizinische Anwendung unabdingbar. Dank des verwirklichten kontrollierten und langsamen Degradationsprozesses entstehen keine oder allenfalls geringe Gasausscheidungen.

Es wurde in vivo und in vitro nachgewiesen, dass die Legierung und Ihre Abbauprodukte äußerst biokompatibel sind. Unter Verwendung der Magnesiumlegierung konnte starken immunologischen Reaktionen des Körpers entgegengewirkt werden. Auch eine kontrolliertes Zellwachstum, insbesondere humaner glatter Muskelzellen und Endothelzellen, konnte anhand von in vitro Versuchen belegt werden. Unkontrollierte Zellwucherungen, die zur Restenose führen können, scheinen verhindert bzw. stark eingedämmt zu sein. Dies ist insbesondere bei der Verwendung von aluminiumhaltigen Legierungen, bei denen starke Neointimahyperplasie beobachtet wurde, derart nicht der Fall. Der den positiven Effekten zugrunde liegende Wirkungsmechanismus ist bisher in Detail nicht ergründet.

Einen Beitrag zur besonderen Verträglichkeit des Implantats könnte Magnesium liefern. Allgemein bekannte Wirkungen und Einflüsse des üblicherweise über die Ernährung aufgenommenen Magnesiums auf die Körperfunktionen lassen vermuten, dass solche Prozesse auch beim Einsatz von Magnesium als Implantat in geeigneter Legierungszusammensetzung zumindest lokal aktiviert werden.

Es ist z. B. bekannt, dass Magnesium im Organismus einen positiven Einfluss auf die Wundheilung hat, da dieses für den anaeroben Stoffwechsel notwendig ist und die normale Granulation des Bindegewebes fördert und unkontrolliertes Zellwachstum eher verhindert (Dr. med. Dr. sc. Nat PG Seeger, SANUM-Post Nr. 13/1990, 14-16).

Ein weiterer positiver Aspekt bei der Verwendung von Magnesium ist, dass die unspezifische Abwehr über das Properdinsystem nur bei Anwesenheit von Magnesium wirksam ist und die Phagozytose von Bakterien durch Leukozyten eine Anregung durch das Magnesium erfährt. Somit sorgt Magnesium u. a. für die Bekämpfung von Infektionen durch Unterstützung bzw. Aktivierung des körpereigenen Immunsystems und mindert die Anfälligkeit für Infektionen. Unerwünschten infektionsbedingten Entzündungserscheinungen aufgrund von Kontaminationen, die im Rahmen der Anwendung einer Endoprothese auftreten können, und die wiederum Auslöser für eine Restenose sein können, wird somit entgegengewirkt.

Die hier verwendete Legierung wirkt auch positiv gegen mechanisch induzierte Restenose. Erreicht wird dies zum einen durch die mechanischen Eigenschaften der verwendeten Legierung, die sich durch ein günstiges Elastizitätsmodul auszeichnen. Des Weiteren wird die allgemein bekannte muskelrelaxierende Wirkung des Magnesium (Ca-Antagonist) genutzt, um mechanische Reizungen zu vermindern. Es ist zu erwarten, dass das Magnesium in der Legierung bzw. die magnesiumhaltigen Zerfallsprodukte bei der Degradation eine Relaxierung der in der näheren Umgebung vorhandenen Muskelzellen fördern. Dies ist insbesondere bei Stent-Anwendungen vorteilhaft, da nicht nur die mechanische Reizung reduziert wird, sondern auch das Gefäß durch das lokal relaxierte Muskelgewebe zusätzlich offen gehalten werden kann.

Die Magnesiumlegierung wird vorzugsweise stranggepresst. Es hat sich herausgestellt, dass die Verarbeitung der Legierung dessen physiologische Wirkung beeinflusst. Diese physiologischen Eigenschaften sind somit zumindest zum Teil durch den Herstellungsprozess bedingt.

Die Endoprothese ist vorzugsweise als intraluminale Endoprothese ausgebildet. Besonders bevorzugt ist eine Endoprothese, die als Stent, insbesondere als Koronarstent oder als peripherer Stent, ausgebildet ist. Aufgrund der positiven Eigenschaften der genannten Magnesiumlegierung besteht die Tragstruktur der Endoprothese vorzugsweise zur Gänze aus der Magnesiumlegierung.

Nach einer bevorzugten Variante für die Anwendung der Legierung als Stent, insbesondere als Koronarstent oder als peripherer Stent, wird die spezifische Zusammensetzung der Magnesiumlegierung sowie deren Modifikation durch die Herstellungsweise und das Stent-Design dahingehend vorgegeben, dass die Zersetzung unmittelbar nach der Implantation einsetzt und die mechanische Integrität für mindestens 5 Tage bis höchstens 1 Jahr aufrecht erhalten wird. Unter "mechanischer Integrität" wird dabei die trotz fortschreitendem Abbau noch ausreichende Stabilität der Strukturelemente des Implantates verstanden, die zur Erfüllung des medizinischen Zwecks des Implantats, d. h. die Aufrechterhaltung der erforderlichen Stützfunktion dienen. Besonders bevorzugt beträgt der Zeitraum 10 bis 90 Tage.

Nachfolgend wird die Erfindung anhand eines Ausführungsbeispiels und dazu gehöriger Zeichnungen näher erläutert. Es zeigen:
- Figur 1 und: eine schematische Darstellung einer Endoprothese in Form eines Stents
- Figur 2: eine Abwicklung der Tragstruktur des Stents aus Figur 1.

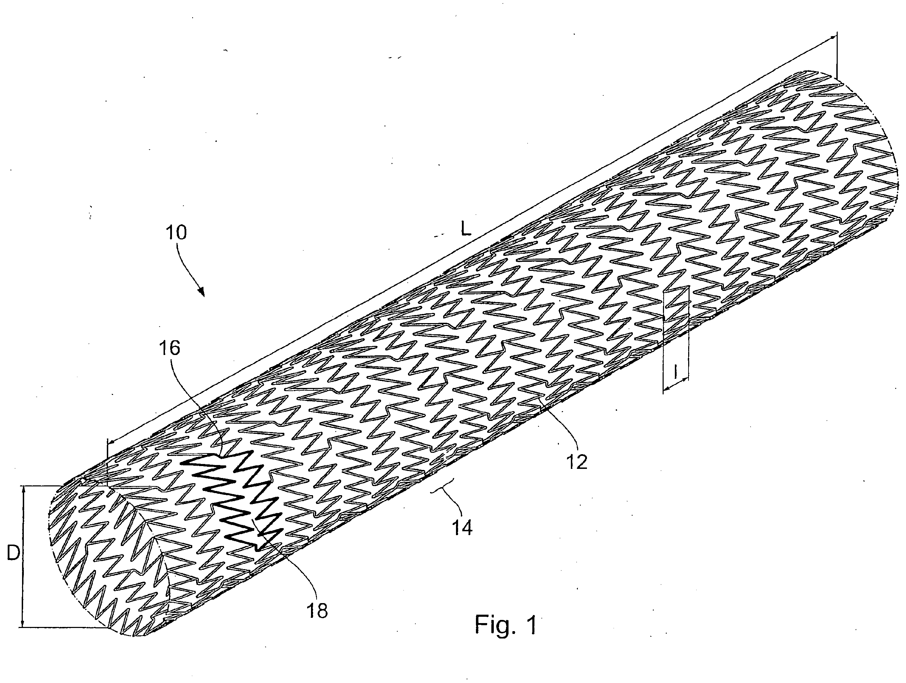

Figur 1 zeigt eine Endoprothese als endoluminale Prothese in Form eines Stents 10 mit einer Tragstruktur. Dieser Stent 10 und seine Tragstruktur haben die Form eines an seinen Stirnseiten offenen Hohlköpers, dessen Umfangswandung von der Tragstruktur gebildet ist, die wiederum von teilweise gefalteten Stegen 12 gebildet ist. Die Stege 12 bilden Stützabschnitte 14, welche von jeweils einem in Längsrichtung ringförmig geschlossenen, zick-zack- oder mäanderförmig gefalteten Steg 12 gebildet sind. Der Stent 10 eignet sich für den koronaren Einsatz.

Die Tragstruktur des Stents 10 wird von mehreren solcher in Längsrichtung aufeinanderfolgender Stützabschnitte 12 gebildet. Die Stützabschnitte 12 bzw. Stegringe 14 sind über Verbindungsstege 16 miteinander verbunden. Jeweils zwei in Umfangsrichtung aneinander benachbarte Verbindungsstege 16 sowie die zwischen diesen Verbindungsstegen 16 einander gegenüberliegenden Teilabschnitte der Stegringe 14 oder Stützabschnitte 12 definieren eine Masche 18 des Stents 10. Eine solche Masche 18 ist in Figur 1 hervorgehoben dargestellt. Jede Masche 18 umschließt eine radiale Öffnung der Umfangswandung bzw. der Tragstruktur des Stents 10.

Jeder Stegring 14 weist etwas drei bis sechs über den Umfang des Stents 10 gleich verteilte Verbindungsstege 16 auf, die jeweils einen Stegring 14 mit dem benachbarten Stegring 14 verbinden. Dementsprechend weist der Stent 10 in Umfangsrichtung zwischen zwei Stützabschnitten 14 jeweils drei bis sechs Maschen 18 auf.

Aufgrund der Faltung der Stege 12 ist der Stent 10 in Umfangsrichtung expandierbar. Dies geschieht beispielsweise mit einem an sich bekannten Ballonkatheter, der an seinem distalen Ende einen mittels eines Fluids expandierbaren Ballon aufweist. Der Stent 10 ist im komprimierten Zustand auf den deflatierten Ballon aufgecrimpt. Mit Expansion des Ballons werden sowohl der Ballon als auch der Stent 10 aufgeweitet. Anschließend kann der Ballon wieder deflatiert werden und der Stent 10 löst sich von dem Ballon. Auf diese Weise kann der Katheter gleichzeitig dem Einführen des Stents 10 in ein Blutgefäß und insbesondere in ein verengtes Herzkranzgefäß sowie zum Expandieren des Stents 10 an diesem Ort dienen.

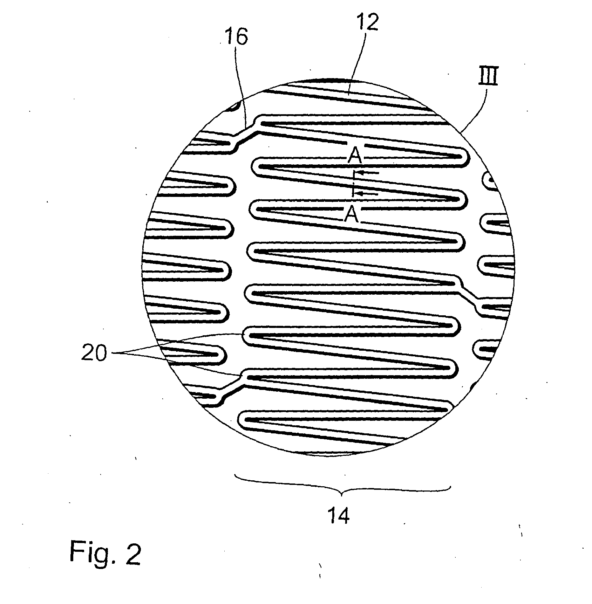

In Figur 2 ist ein Ausschnitt aus einer Abwicklung der Umfangswandung des Stents 10 dargestellt. Die Abwicklung zeigt den komprimierten Zustand des Stents 10.

Die Tragstruktur des in den Figuren dargestellten Stents 10 besteht vollständig aus einer biodegradierbaren Magnesiumlegierung folgender Zusammensetzung:
Magnesium 65 Gew.%,
Seltenerdmetalle 15.0 Gew.%, dabei Neodym 2.3 Gew.%,
Yttrium 10.0 Gew.%,
Zirkonium 3.0 Gew.%,
Lithium 3.0 Gew.%,
Eisen 2.0 Gew.%,
Zink 2.0 Gew.%,
Aluminium, Silber, Kupfer, Quecksilber, Cadmium, Beryllium, Chrom und Nickel < 0.005 Gew.%*, (*Nachweisgrenze bei der Bestimmung)
wobei sich die Legierungskomponenten auf 100 Gew.% addieren.

## Patentansprüche

1. Endoprothese mit einer Tragstruktur, die ganz oder in Teilen aus einer Magnesiumlegierung folgender Zusammensetzung besteht:
· Magnesium 60.0 bis 88.0 Gew.%,
· Seltenerdmetalle 2.0 bis 30.0 Gew.%,
· Yttrium 2.0 bis 20.0 Gew.%,
· Zirkonium 0.5 bis 5.0 Gew.%,
· Rest 0 bis 10.0 Gew.%,
wobei sich die Legierungskomponenten auf 100 Gew.% addieren.

2. Endoprothese nach Anspruch 1, **dadurch gekennzeichnet, dass** der Anteil von Magnesium an der Legierung 60.0 bis 70.0 Gew.% beträgt.

3. Endoprothese nach Anspruch 1, **dadurch gekennzeichnet, dass** der Anteil an Neodym 0.5 Gew.% bis 10.0 Gew.% beträgt.

4. Endoprothese nach Anspruch 3, **dadurch gekennzeichnet, dass** der Anteil an Neodym 2.0 Gew.% bis 2.5 Gew.% beträgt.

5. Endoprothese nach Anspruch 1, **dadurch gekennzeichnet, dass** der Rest eines oder mehrere Elemente aus der Gruppe Lithium, Zink, Eisen, Titan, Tantal, Molybdän und Wolfram enthält.

6. Endoprothese nach Anspruch 1, **dadurch gekennzeichnet, dass** der Rest kein oder allenfalls < 0.01 Gew.% Aluminium enthält.

7. Endoprothese nach Anspruch 1, **dadurch gekennzeichnet, dass** der Rest kein oder allenfalls < 0.03 Gew.% Kupfer enthält.

8. Endoprothese nach Anspruch 1, **dadurch gekennzeichnet, dass** der Rest kein oder allenfalls < 0.005 Gew.% Nickel enthält.

9. Endoprothese nach Anspruch 1, **dadurch gekennzeichnet, dass** der Rest kein oder allenfalls < 0.01 Gew.% Silber enthält.

10. Endoprothese nach Anspruch 1, **dadurch gekennzeichnet, dass** der Rest kein oder allenfalls < 0.03 Gew.% Quecksilber enthält.

11. Endoprothese nach Anspruch 1, **dadurch gekennzeichnet, dass** der Rest kein oder allenfalls < 0.03 Gew.% Cadmium enthält.

12. Endoprothese nach Anspruch 1, **dadurch gekennzeichnet, dass** der Rest kein oder allenfalls < 0.03 Gew.% Chrom enthält.

13. Endoprothese nach Anspruch 1, **dadurch gekennzeichnet, dass** der Rest kein oder allenfalls < 0.03 Gew.% Beryllium enthält.

14. Endoprothese nach einem der vorhergehenden Ansprüche, **dadurch gekennzeichnet, dass** die Endoprothese als intraluminale Endoprothese ausgebildet ist.

15. Endoprothese nach Anspruch 14, **dadurch gekennzeichnet, dass** die Endoprothese als Stent ausgebildet ist.

16. Endoprothese nach Anspruch 1, **dadurch gekennzeichnet, dass** die Seltenerdmetalle 2.0 bis 30.0 Gew.% Gadolinium enthalten.

## Claims

1. An endoprosthesis having a supporting structure, consisting entirely or in part of a magnesium alloy having the following composition:
- magnesium 60.0 to 88.0 wt%,
- rare earth metals 2.0 to 30.0 wt%,
- yttrium 2.0 to 20.0 wt%,
- zirconium 0.5 to 5.0 wt%,
- remainder 0 to 10.0 wt%,
wherein the alloy components add up to 100 wt%.

2. The endoprosthesis according to Claim 1, **characterized in that** the amount of magnesium in the alloy is 60.0 wt% to 70.0 wt%.

3. The endoprosthesis according to Claim 1, **characterized in that** the amount of neodymium is 0.5 wt% to 10.0 wt%.

4. The endoprosthesis according to Claim 3, **characterized in that** the amount of neodymium is 2.0 wt% to 2.5 wt%.

5. The endoprosthesis according to Claim 1, **characterized in that** the remainder contains one or more elements from the group of lithium, zinc, iron, titanium, tantalum, molybdenum and tungsten.

6. The endoprosthesis according to Claim 1, **characterized** that the remainder contains none or at most <0.01 wt% aluminum.

7. The endoprosthesis according to Claim 1, **characterized** that the remainder contains none or at most <0.03 wt% copper.

8. The endoprosthesis according to Claim 1, **characterized** that the remainder contains none or at most <0.005 wt% nickel.

9. The endoprosthesis according to Claim 1, **characterized** that the remainder contains none or at most <0.01 wt% silver.

10. The endoprosthesis according to Claim 1, **characterized** that the remainder contains none or at most <0.03 wt% mercury.

11. The endoprosthesis according to Claim 1, **characterized** that the remainder contains none or at most <0.03 wt% cadmium.

12. The endoprosthesis according to Claim 1, **characterized** that the remainder contains none or at most <0.03 wt% chromium.

13. The endoprosthesis according to Claim 1, **characterized** that the remainder contains none or at most <0.03 wt% beryllium.

14. The endoprosthesis according to any one of the preceding claims, **characterized in that** the endoprosthesis is designed as an intraluminal endoprosthesis.

15. The endoprosthesis according to Claim 14, **characterized in that** the endoprosthesis is designed as a stent.

16. The endoprosthesis according to Claim 1, **characterized in that** the rare earth metals contain 2.0 wt% to 30.0 wt% gadolinium.

## Revendications

1. Endoprothèse avec une structure portante, qui est tout ou en partie à base d'un alliage de magnésium de la composition suivante :
• magnésium 60,0 jusqu'à 88,0 % en poids,
• métaux des terres rares 2,0 jusqu'à 30,0 % en poids,
• yttrium 2,0 jusqu'à 20,0 % en poids,
• zirconium 0,5 jusqu'à 5,0 % en poids
• le reste 0 à jusqu'à 10,0 % en poids,
les composants d'alliage s'accumulant jusqu'à 100 % en poids.

2. Endoprothèse selon la revendication 1, **caractérisée en ce que** la fraction de magnésium dans l'alliage va de 60,0 jusqu'à 70,0 % en poids.

3. Endoprothèse selon la revendication 1, **caractérisée en ce que** la fraction de néodyme est de 0,5 % en poids jusqu'à 10,0 % en poids.

4. Endoprothèse selon la revendication 3, **caractérisée en ce que** la fraction de néodyme va de 2,0 % en poids jusqu'à 2,5 % en poids.

5. Endoprothèse selon la revendication 1, **caractérisé en ce que** le reste contient un ou plusieurs éléments provenant du groupe lithium, zinc, fer, titane, tantale, molybdène et tungstène.

6. Endoprothèse selon la revendication 1, **caractérisée en ce que** le reste ne contient pas ou dans tous les cas moins de 0,01 % en poids d'aluminium.

7. Endoprothèse selon la revendication 1, **caractérisée en ce que** le reste ne contient pas ou dans tous les cas moins de 0,03 % en poids de cuivre.

8. Endoprothèse selon la revendication 1, **caractérisée en ce que** le reste ne contient pas ou dans tous les cas moins de 0,005 % en poids de nickel.

9. Endoprothèse selon la revendication 1, **caractérisée en ce que** le reste ne contient pas ou dans tous les cas moins de 0,01 % en poids d'argent.

10. Endoprothèse selon la revendication 1, **caractérisée en ce que** le reste ne contient pas ou dans tous les cas moins de 0,03 % en poids de mercure.

11. Endoprothèse selon la revendication 1, **caractérisée en ce que** le reste ne contient pas ou dans tous les cas moins de 0,03 % en poids de cadmium.

12. Endoprothèse selon la revendication 1, **caractérisée en ce que** le reste ne contient pas ou dans tous les cas moins de 0,03 % en poids de chrome.

13. Endoprothèse selon la revendication 1, **caractérisée en ce que** le reste ne contient pas ou dans tous les cas moins de 0,03 % en poids de béryllium.

14. Endoprothèse selon une des revendications précédentes, **caractérisée en ce que** l'endoprothèse est conçue comme endoprothèse intraluminaire.

15. Endoprothèse selon la revendication 14, **caractérisée en ce que** l'endoprothèse est conçue comme stent.

16. Endoprothèse selon la revendication 1, **caractérisée en ce que** les métaux de terres rares contiennent 2,0 jusqu'à 30,0 % en poids de gadolinium.
